# EUROPEAN PATENT SPECIFICATION

(11) **EP 1 583 969 B1**
(45) Date of publication and mention of the grant of the patent: **02.11.2006**
(21) Application number: 03782366.3
(22) Date of filing: 05.12.2003
(51) Int. Cl.: G01N 33/68

(54) **METHOD FOR SCREENING FOR AGENTS PREVENTING AGGREGATION OF POTENTIALLY AMYLOIDOGENIC PROTEINS**
VERFAHREN ZUR IDENTIFIZIERUNG VON VERBINDUNGEN, WELCHE DIE AGGREGATION VON AMYLOIDOGENEN PROTEINEN VERHINDERN KÖNNEN
PROCEDE DE CRIBLAGE D'AGENTS EMPECHANT L'AGREGATION DE PROTEINES POTENTIELLEMENT AMYLOIDOGENIQUES

(30) Priority: 09.12.2002 IT MI20022607
(43) Date of publication of application: 12.10.2005
(73) Proprietor: Tortora, Paolo, 20135 Milano (IT); Fusi, Paola, 20146 Milano (IT); Shehi, Erlet, 20158 Milano (IT)
(72) Inventor: Tortora, Paolo, 20135 Milano (IT); Fusi, Paola, 20146 Milano (IT); Shehi, Erlet, 20158 Milano (IT)
(74) Representative: Pistolesi, Roberto
(86) International application number: PCT/EP2003/014047
(87) International publication number: WO 2004/053484

(56) References cited:
- WO-A-01/06989
- WO-A-97/26919
- WO-A-02/064618
- WO-A-02/064619
- WO-A-02/074931
- US-B1- 6 420 122
- UVERSKY VLADIMIR N ET AL: "Stabilization of partially folded conformation during alpha-synuclein oligomerization in both purified and cytosolic preparations" JOURNAL OF BIOLOGICAL CHEMISTRY, vol. 276, no. 47, 23 November 2001 (2001-11-23), pages 43495-43498, XP002281257 ISSN: 0021-9258
- KUSUMOTO YOKO ET AL: "Temperature dependence of amyloid beta-protein fibrillization" PROCEEDINGS OF THE NATIONAL ACADEMY OF SCIENCES OF THE UNITED STATES OF AMERICA, vol. 95, no. 21, 13 October 1998 (1998-10-13), pages 12277-12282, XP002281249 ISSN: 0027-8424
- HASHIMOTO MAKOTO ET AL: "Human recombinant NACP/alpha-synuclein in aggregated and fibrillated in vitro: Relevance for Lewy body disease" BRAIN RESEARCH, vol. 799, no. 2, 20 July 1998 (1998-07-20), pages 301-306, XP002943678 ISSN: 0006-8993

## Description

The invention provides a method for screening compounds capable of preventing aggregation of amyloidogenic proteins and peptides. Such compounds are potential drugs for the treatment of polyglutamine diseases, and possibly of other related pathologies such as Alzheimer's and prion diseases.

### FIELD OF THE INVENTION

Some human proteins display in their sequence tracts of consecutive polyglutamines, usually not exceeding 40 residues. In some individuals, however, these repeats occur in expanded form, i.e., they consist of higher than 40 and up to more than 100 residues. As a consequence, these individuals suffer from neurodegenerative diseases, referred to as polyglutamine (or poly-Q) diseases [Perutz, M.F. (1999) Trends Biochem. Sci., 24, 58-63]. Known poly-Q diseases are Huntington disease (HD), spinobulbar muscular atrophy (SBMA), dentatorubropallidoluysian atrophy (DRPLA), spinocerebellar ataxia type 1 (SCA1), SCA2, SCA3, SCA6, SCA7 [Gusella, J.F. & MacDonald, M.E. (2000) Nature Neurosci. 1, 109-115]. In each of these diseases a different protein is involved, no sequence and evolutionary relationship being evident among them. The frequency of these diseases ranges from 1:10,000 to 1:100000. The underlying genetic mechanism results from glutamine being coded in genes by the triplets CAG and CAA. So poly-Qs are often encoded by CAG repeats. Such repeats are unstable and, for reasons still poorly understood, in some familial lines tend to increase in size, thus giving rise to expanded poly-Q stretches in the proteins involved [Zoghbi, H.Y. & Horr., H.T. (2000) Annu. Rev. Neurosci. 23, 217-247].

These diseases are similar to each other in terms of clinical, neuropathological and molecular features. So it is generally accepted that they also share the basic pathogenetic mechanisms, which are accounted for by the effects of poly-Q stretches at the molecular level, irrespective of the host protein. At the molecular level, it has been also observed that such diseases are paralleled by poly-Q-triggered appearance of neuronal aggregates, mostly nuclear, which also recruit several other non-poly-Q proteins. This results eventually in loss of protein function. Although the causal relationship among the events leading to neuronal death is far from being completely clarified, it is well established that poly-Q aggregation is the earliest event, which triggers all the subsequent [Perutz. M.F. & Windle, A.H. (2001) Nature 412, 143-144]. This justifies the remarkable effort put in developing drugs capable of preventing these diseases by just hampering the aggregation process. Furthermore, the characterisation of aggregates generated by proteins carrying expanded poly-Q stretches has clearly shown that they must be regarded as amyloid fibres with respect to morphology and reactivity towards specific compounds (in particular Congo red) [Perutz; M.F. et al. (2002) Proc. Natl. Acad. Sci. USA 99,5591-5595]. Amyloid fibres also occur in other neurodegenerative pathologies, notably Alzheimer's and prion diseases. Therefore, it is expected that compounds capable of preventing the aggregation of proteins carrying expanded poly-Qs, will be also effective in treating the aforementioned diseases.

### DESCRIPTION OF THE INVENTION

The present patent deals with a new *in vitro* procedure for screening compounds capable of preventing aggregation, either among amyloidogenic proteins or between an amyloidogenic protein and any other protein. In particular, amyloidogenic proteins of interest may be poly-Q proteins, although many others are actually amyloidogenic although devoid of any poly-Q stretch. These are, for instance, beta-amyloid and prion proteins.

Known *in vitro* screening procedures involve the adoption of two different proteins prone to aggregation in that they contain expanded poly-Q or other aggregation-disposed stretches. Such proteins may be beta-amyloid peptides, tau proteins, alpha synucleins and prion proteins. Procedures essentially consist in mixing two peptides of this type (either identical or different), whilst one of them is covalently bound to another protein acting as a detection system. This may be either a fluorescent protein or an enzyme. The basic principle requires that the signal detected (either fluorescence or enzyme activity) is changed as a result of aggregation. This makes it possible to demonstrate the antiaggregating effect of a compound with respect to the control assay, wherein no compound is present. Such procedures may also encompass addition of a denaturing agent to the mixture after the peptides have been mixed. In such case, it is expected that the aggregate arising from the interaction between the two peptides is able to withstand the denaturant. So the denaturant either reduces or abolishes the activity of the detection system (either fluorescent protein or enzyme) only provided that the aggregation has been prevented. Under such conditions, a compound preventing aggregation also leads to reduction or suppression of the signal of the detection system with respect to the control sample, wherein such compound is absent. For instance, these approaches are reported in US 6,420,122 B1 and in WO 02/064618.

The present invention relies instead upon the following principles: a) the availability of a protein soluble in a temperature range whose lower limit is 0°C and whose upper limit is comprised between 50°C and 80°C b) said protein must be able to generate amyloid fibres if heated up to temperatures higher than the mentioned upper limit c) said protein must not generate amorphous aggregates when heated under the conditions described in b).

The screening procedure encompasses: mixing of the protein of interest with the compound to be assayed; progressive temperature increasing; monitoring of the appearance of amyloid fibres by turbidimetric measurements, and/or by visual inspection of the mixture, and/or by determining its protein content after spinning down the aggregated protein. In the control sample, where the compound of interest is absent, heating will lead to amyloid fibres formation and the subsequent clouding of the solution. In contrast, a compound capable of preventing aggregation, will prevent or reduce clouding and protein precipitation on sample centrifugation.

Proteins suitable for the present invention are polyglutaminated proteins carrying a sequence of consecutive glutamines that enable the host proteins to give rise to amyloid fibres on heating. Preferably, the sequence length will be 32 residues or more, even more preferably, the protein will be an ataxin-3 variant.

The invention encompasses the development of a suitable expression system, consisting of a plasmid carrying the protein-encoding gene. The protein may be expressed either in authentic form, or as a fusion protein with suitable fusion partners. They may be preferably, but not necessarily: a) a polyhistidine stretch b) glutathione S-transferase. If the protein is expressed as a fusion protein, a cleavage site for a specific protease may be inserted between the fusion partner and the polyglutaminated protein. The protein is expressed as a heterologous protein in suitable organisms, preferably but not necessarily *Escherichia coli* or yeast. The invention also encompasses the development of suitable protein purification procedures, either following conventional methods of protein chemistry, or adopting matrices for affinity chromatography, which selectively bind the fusion partner. Subsequently, the fusion partner may be either removed by a specific protease, or retained. In the latter case the whole protein construct will be used, provided it is able to give rise to amyloid fibres on heating, and not to amorphous aggregates.

### DETAILED DESCRIPTION OF THE INVENTION

The invention relies upon a simple procedure to identify compounds endowed with the ability to prevent the aggregation of amyloids-generating proteins. Such aggregation events underlie several neurodegenerative diseases, specifically poly-Q diseases (such as Huntington's corea), Alzheimer's disease, Parkinson's disease, prion diseases. Thus, a compound counteracting these aggregation events is a potential drug for the prevention of these diseases.

Such amyloids-generating proteins or polypeptides may be natural or non-natural, may contain a polyglutamine tract, either continuous or broken by interspersed amino acids other than glutamine, preferably but not necessarily lysine. Alternatively, these proteins may be natural amyloidogenic non-poly-Q proteins, or their variants.

The essential requirement for these proteins is their capability to generate amyloid fibres, but not amorphous aggregates, on heating. In other words, they must withstand relatively high temperatures, for instance higher than 50°C (unlike most proteins form animal sources), without undergoing denaturation and the ensuing formation of amorphous aggregates.

In contrast, these proteins must be soluble in aqueous solutions below a given temperature value but must generate amyloid fibres as a result of heating above this value. Said value is preferably between 40°C and 90°C, and more preferably between 55° and 85°C:

In case the amyloidogenic protein is a polyglutaminated protein, it must carry a poly-Q sequence capable to generate amyloid fibres on heating, as mentioned above. The length of such sequence is between 32 and 300 glutamines, preferably between 32 and 100, more preferably between 32 and 52.

The invention also encompasses that the polyglutamine tract of the aforementioned length be broken by one or few interspersed amino acids other than glutamine, preferably but not necessarily lysine. Such breaks may consist of one ore more sequences of 1 to 3 amino acids other than glutamine, preferably of single amino acids, more preferably of only one amino acid (which, in the best exploitation of the invention, is a lysine).

The gene sequence coding for the polyglutamine tract may contain CAG codons only, or CAA codons only, or also a mixture of both, not necessarily in equal number.

Natural proteins potentially endowed with the mentioned properties are: huntingtin (causative agent of Huntington's corea); ataxin-1 (causative agent of spinocerebellar ataxia type 1, or SCA1), ataxin-2 (causative agent of spinocerebellar ataxia type 2, or SCA2), ataxin-3 (causative agent of spinocerebellar ataxia type 3, or SCA3), voltage-gated alpha 1a calcium channel (causative agent of spinocerebellar ataxia type 6, or SCA6), ataxin-7 (causative agent of spinocerebellar ataxia type 7, or SCA7), androgen receptor (causative agent of spinobulbar muscular atrophy, or SBMA). Other aggregation-disposed proteins are synucleins (involved in Alzheimer's and Parkinson's diseases), beta-amyloids (involved in Alzheimer's diseases), prion proteins (involved in spongiform encephalopathies). The invention includes said proteins, provided they fulfil the aforementioned requirements.

Non-natural, aggregation-disposed, amyloid-forming proteins are: variants of all aforementioned proteins, as well as peptides not found in biological systems (for instance, isolated tracts of poly-Q, or poly-Q tracts carrying amino acids at the N- and C-termini) provided they fulfil the aforementioned requirements. Non-natural proteins produced as variants of natural proteins, must either share a high sequence identity with one of the aforementioned natural proteins (for instance, 60% to 99%), or represent a fragment of the above proteins. The invention includes said proteins provided they fulfil the above-described requirements.

### Expression systems, vectors and protein-encoding genes

Vectors and expression systems to be employed for producing the proteins of interest are commercially available and well known in the art. Host cells may be *Escherichia coli,* yeast, or any other microorganism in which the proteins of interest are mostly soluble under practicable growth conditions. Vectors may be those commercially available, which allow inducible expression of the protein (using, for instance, isopropyl beta thiogalactoside as an inducer). Proteins of interest may be expressed either in authentic form, or as a fusion protein with suitable partners. They may be preferably, but not necessarily: a) a polyhistidine stretch b) glutathione S-transferase. If the protein is expressed as a fusion protein, a cleavage site for a specific protease may be inserted between the fusion partner and the polyglutaminated protein.

Genes expressing the proteins of interest may be produced either following standard cloning procedures well known in the art, or by automated chemical synthesis adopting standard techniques. Gene sequences coding for polyglutammine may contain CAG codons only, or CAA codons only, or also a mixture of both, not necessarily in equal number.

### Purification of proteins or polypeptides to be employed in the invention

The development of the screening system the present invention deals with, requires the exploitation of purified amyloidogenic proteins or peptides. Purification may be carried out by adopting traditional procedures of protein chemistry, well known in the art. If the protein is expressed as a fusion protein, a purification step will be adopted taking advantage of the specific recognition of the fusion partner by suitable matrices for affinity chromatography. The fusion partner may be then either removed by specific proteases, or retained. In the latter case the whole protein construct will be used, provided it is able to give rise to amyloid fibres on heating, and hot to amorphous aggregates.

### Screening assays and identification of compounds preventing aggregation of amyloidogenic proteins

Screening assays are based on the availability of a protein which is: a) soluble in a given temperature range b) able to give rise to amyloid fibres at temperatures above the mentioned range c) thermostable to such an extent that it can withstand heating without undergoing denaturation and ensuing formation of amorphous aggregates at any temperature required for amyloid fibre formation.

Thus, in this screening system, precipitation only results from intermolecular interactions among protein regions capable of forming amyloid fibres, typically poly-Q tracts.

The screening procedure encompasses a) mixing the protein of interest with the compound to be assayed for its ability to prevent aggregation b) gradually increasing the temperature c) detecting the appearance of amyloid fibres by turbidimetric measurements, and/or by visual inspection of the mixture, and/or by determining its protein content before and after spinning down the aggregated protein. In the control sample, no compound is added so heating results in amyloid fibre formation and ensuing protein precipitation and clouding of the solution. In contrast, a compound capable of preventing aggregation also prevents or reduces clouding, along with protein precipitation on centrifugation. Protein concentration is typically about 0.1 mg per ml.

A preliminary selection of compounds to be assayed may be accomplished on the basis of theoretical principles. For instance, based on the notion that aggregation requires hydrogen bonding between poly-Q stretches, compounds competing for hydrogen bonds may be assayed. Alternatively, a fully random screening may be carried out by assaying whole chemical libraries. This is feasible thanks to the simplicity of the system here described, which makes it possible to develop a high-throughput screening whereby, in principle, any chemical can be assayed. However, in view of the intended clinical applications of compounds proving to be potentially effective, preferred candidates will be small-sized molecules (for instance, peptides or small organic molecules).

### EXAMPLES

### Example 1 - Development of an expression system for producing human ataxin-3 (Q26) and human ataxin-3 (Q36)

cDNA coding for human ataxin-3 (Q26) was constructed by assembling the C-terminal coding sequence contained in IMAGE clone 79914 and the N-terminal coding sequence found in IMAGE clone number 4393766 (both purchased from the RZPD Deutsches Resourcenzentrum für Genomforschung GmbH). The assembled sequence was then subcloned into plasmid pGEX6P-1. This plasmid allows cloning of foreign genes under an isopropyl beta-D-thiogalactopyranoside-inducible tac promoter. Recombinant proteins can be expressed as glutathione S-transferase fusion proteins, and retrieved by means of a PreScission Protease (Amersham Biosciences UK Ltd., Little Chalfont, England) cleavage site located in between the two coding sequences. Human ataxin-3 (Q36) was produced by growing, on LB-Ampicillin medium plates, *Escherichia coli* cells (strain BL21 codon plus RIL) carrying human ataxin-3 (Q26) gene inserted in pGEX6P-1. Human ataxin-3 carries upstream of the 36 consecutive glutamines, one lysine and three more glutamines.

### Example 2 - Purification of human ataxin-3 (Q26) and human ataxin-3 (Q36)

Human ataxins-3 were expressed in *Escherichia coli* strain BL21 codon plus RIL. Encoding genes were inserted in pGEX6P-1, and coded for fusion proteins with glutathione S-transferase, with a cleavage site for "Prescission Protease" between ataxins-3 and glutathione S-transferase. Cells were grown at 37°C in LB-ampicillin medium and induced with 50 µM IPTG at A₆₀₀ = 1.0 for 2 h. About 13 g cells were pelleted from 4 liter of culture. To obtain crude extracts, cells were frozen and thawed, then incubated for 1 h at 4°C in 100 ml of lysis buffer (10 mM sodium phosphate, pH 7.2, 150 mM NaCl, 1mM phenylmethanesulfonyl fluoride, 10 mM dithiothreitol, 100 mM MgCl₂, 0.5 mg/ml lysozyme), and again frozen and thawed. 1 % Triton-X-100 and DNase (0.2 mg/g cells) were then added and the samples further incubated for 30 min at room temperature. Finally, they were centrifuged for 30 min at 47000 x g. The supematants were loaded onto a Glutathione Sepharose 4B affinity column (3 ml bed volume). The column was washed with three volumes of 10 mM sodium phosphate, pH 7.2, 150 mM NaCl, followed by three volumes of cleavage buffer (50 mM Tris-HCl, pH 7.0, 150 mM NaCl, 1 mM EDTA, 1 mM DTT). Removal of the glutathione S-transferase affinity tail was achieved by incubating overnight at 4°C the resin-bound proteins in the presence of PreScission Protease (400 U/ml resin). Mature ataxins-3 were then eluted with cleavage buffer, while PreScission Protease, being a glutathione S-transferase fusion protein, remained bound to the resin. Ataxins-3 both Q26 and Q36, were subsequently concentrated in Centricon YM-30 microconcentrators (Millipore Corporation, Bedford, MA) to final concentrations of 2-5 mg/ml.

### Example 3 - On heating, human ataxin-3 (Q36) gives rise to amyloid fibres

Human ataxin-3 (Q36) was dissolved in 10 mM sodium phosphate, pH 7.0 at a final concentration of about 0.1 mg/ml and incubated at growing temperatures. Circular dichroism measurements showed that, starting form 40°C, the protein underwent a progressive decrease in alpha-helix content along with a progressive increase in beta-sheet. At about 80°C, the protein showed tendency to aggregate, which resulted in clouding of the solution and formation of precipitates.

In contrast, ataxin-3 (Q26) was stable almost up to the boiling point, as shown by the lack of significant spectral changes in circular dichroism and the absence of any protein precipitation.

Precipitates formed on heating ataxin-3 (Q36), displayed the typical properties of amyloid fibres, as supported by the following: evidences: a) their morphological features, shown in electron microscopy b) the characteristic red shift of Congo red spectra, which is regarded as diagnostic of a specific interaction of the dye with amyloid fibres. Such shift was more and more pronounced as the temperature was increased in the range 55°C to 85°C c) the reactivity towards 1C2 monoclonal antibodies, which selectively recognise expanded, amyloidogenic poly-Q tracts, but not those of normal length.

### Example 4 - Congo red prevents amyloid fibre formation

Congo red is a well-known antiamyloidogenic compound. On incubating human ataxin-3 (Q36) in the presence of 10 microM Congo red and otherwise under the conditions depicted in example 3, protein aggregation phenomena observed in the absence of the dye were prevented. This shows that the screening system is suitable for selecting potentially antiamyloidogenic compounds.

## Claims

1. A method for identifying compounds preventing the aggregation of amyloidogenic proteins or peptides comprising the following steps: (a) preparing an aqueous solution containing the compound to be assayed and a protein carrying a polyglutamine sequence of at least 32 residues; (b) increasing the temperature of the solution up to a value equal to or higher than that causing said protein to form amyloid fibres and (c) checking the occurrence of amyloid fibres; said method being **characterized in that** the temperature of step (b) is comprised between 55°C and 90°C and **in that** the appearance of amyloid fibres is checked by turbidimetric measurements.

2. The method of claim 1, wherein said protein contains a sequence of 32-300 glutamines, preferably of 32-100 glutamines, more preferably 32-52 glutamines.

3. The method of claim 1, wherein said protein contains a sequence of 36 glutamines.

4. The method of claims 1-3, wherein said glutamine sequence contains one or more interspersed amino acids other than glutamine.

5. The method of claim 4, wherein said glutamine sequence contains one or more interspersed sequences of 1-3 amino acids other than glutamine.

6. The method of claim 4, wherein said glutamine sequence contains one amino acid other than glutamine.

7. The method of claims 4-6, wherein said protein contains a sequence of 39 glutamines which, in turn, contains one amino acid other than glutamine.

8. The method of claims 4-7, wherein said amino acid other than glutamine is lysine.

9. The method of claims 1-8, wherein said temperature value is comprised between 55°C and 85°C.

10. The method of claim 9, wherein said temperature value is about 80°C.

11. The method of claims 1-10, wherein said protein is selected between: huntingtin, atrophin-1, ataxin-1, ataxin-2, ataxin-3, ataxin-7, alpha 1a calcium channel, androgen receptor, or is a non-natural protein, consisting of a polyglutamine stretch made soluble by the presence of charged amino acids at the two ends.

12. The method of claims 1-10, wherein said protein is selected between: tau protein synuclein, beta-amyloid, prion protein, albeit devoid of poly-glutamines.

13. The method according to claims 1-12 wherein the occurrence of amyloid fibres is checked by visual inspection of the mixture and/or by determining its protein content after spinning down the aggregated protein.

## Patentansprüche

1. Verfahren zur Identifizierung von Verbindungen, welche die Aggregation von amyloidogenen Proteinen oder Peptiden verhindern, umfassend die folgenden Schritte:
(a) Zubereiten einer wässrigen Lösung, enthaltend die zu untersuchende Verbindung und ein Protein, welches eine Polyglutamin-Sequenz mit mindestens 32 Resten trägt;
(b) Erhöhen der Temperatur der Lösung auf einen Wert, der dem Wert entspricht oder höher ist als der Wert, bei dem das Protein Amyloidfasern bildet und
(c) Überprüfen, ob Amyloidfasern auftreten;
wobei das Verfahren **dadurch gekennzeichnet ist, dass** die Temperatur von Schritt (b) zwischen 55°C und 90°C liegt und dass das Auftreten von Amyloidfasern durch turbidimetrische Messungen überprüft wird.

2. Verfahren nach Anspruch 1, wobei das Protein eine Sequenz von 32 - 300 Glutaminen, bevorzugt von 32 - 100 Glutaminen, mehr bevorzugt von 32 - 52 Glutaminen enthält.

3. Verfahren nach Anspruch 1, wobei das Protein eine Sequenz von 36 Glutaminen enthält.

4. Verfahren nach den Ansprüchen 1 - 3, wobei die Glutamin-Sequenz eine oder mehrere dazwischenliegende Aminosäuren enthält, die nicht Glutamin sind.

5. Verfahren nach Anspruch 4, wobei die Glutamin-Sequenz eine oder mehrere dazwischenliegende Sequenzen von 1 - 3 Aminosäuren, die nicht Glutamin sind, enthält.

6. Verfahren nach Anspruch 4, wobei die Glutamin-Sequenz eine Aminosäure, die nicht Glutamin ist, enthält.

7. Verfahren nach den Ansprüchen 4-6, wobei das Protein eine Sequenz von 39 Glutaminen enthält, welche wiederum eine Aminosäure, die nicht Glutamin ist, enthält.

8. Verfahren nach den Ansprüchen 4-7, wobei die Aminosäure, die nicht Glutamin ist, Lysin ist.

9. Verfahren nach den Ansprüchen 1 - 8, wobei der Temperaturwert zwischen 55°C und 85°C liegt.

10. Verfahren nach Anspruch 9, wobei der Temperaturwert ungefähr 80°C beträgt.

11. Verfahren nach den Ansprüchen 1 - 10, wobei das Protein ausgewählt wird aus:
Huntingtin, Atrophin-1, Ataxin-1, Ataxin-2, Ataxin-3, Ataxin-7, alpha 1a Kalzium Kanal, Androgenrezeptor, oder ein nicht-natürliches Protein ist, das aus einem durch die Anwesenheit von geladenen Aminosäuren an den zwei Enden löslich gemachten Polyglutamin-Abschnitt besteht.

12. Verfahren nach den Ansprüchen 1 - 10, wobei das Protein ausgewählt wird aus: Tau-Protein Synuclein, beta-Amyloid, Prion Protein, wenngleich frei von Poly-Glutaminen.

13. Verfahren nach den Ansprüchen 1 - 12, wobei das Auftreten von Amyloidfasern, durch visuelle Inspektion des Gemisches und/oder durch Bestimmung seines Proteingehalts nach dem Abzentrifugieren des aggregierten Proteins überprüft wird.

## Revendications

1. Procédé pour identifier des composés prévenant l'agrégation de protéines ou peptides amyloïdogéniques, comprenant les étapes suivantes: (a) préparer une solution aqueuse contenant le composé à tester et une protéine portant une séquence polyglutamine d'au moins 32 résidus; (b) augmenter la température de la solution jusqu'à une valeur égale ou supérieure à celle qui provoque la formation de fibres amyloïdes par ladite protéine et (c) vérifier la formation de fibres amyloïdes; ledit procédé étant **caractérisé en ce que** la température de l'étape (b) est comprise entre 55°C et 90°C et **en ce que** l'apparition de fibres amyloïdes est vérifiée par mesures de turbidimétrie.

2. Procédé selon la revendication 1, dans lequel ladite protéine contient une séquence de 32-300 glutamines, de préférence de 32-100 glutamines, plus préférentiellement de 32-52 glutamines.

3. Procédé selon la revendication 1, dans lequel ladite protéine contient une séquence de 36 glutamines.

4. Procédé selon l'une des revendications 1-3, dans lequel ladite séquence de glutamines contient un ou plusieurs acides aminés intercalés autres que la glutamine.

5. Procédé selon la revendication 4, dans lequel ladite séquence de glutamines contient une ou plusieurs séquences intercalées de 1-3 acides aminés autres que la glutamine.

6. Procédé selon la revendication 4, dans lequel ladite séquence de glutamines contient un acide aminé autre que la glutamine.

7. Procédé selon l'une des revendications 4-6, dans lequel ladite protéine contient une séquence de 39 glutamines qui contient elle-même un acide aminé autre que la glutamine.

8. Procédé selon l'une des revendications 4-7, dans lequel ledit acide aminé autre que la glutamine est la lysine.

9. Procédé selon l'une des revendications 1-8, dans lequel ladite valeur de température est comprise entre 55°C et 85°C.

10. Procédé selon la revendication 9, dans lequel ladite valeur de température est 80°C environ.

11. Procédé selon l'une des revendications 1-10, dans lequel ladite protéine est choisie parmi: la huntingtine, l'atrophine-1, l'ataxine-1, l'ataxine-2, l'ataxine-3, l'ataxine-7, le canal calcique alpha 1a, le récepteur aux androgènes, ou est une protéine non-naturelle constituée d'une série polyglutamine rendue soluble par la présence d'acides aminés chargés aux deux extrémités.

12. Procédé selon l'une des revendications 1-10, dans lequel ladite protéine est choisie parmi: la protéine tau, la synucléine, l'amyloïde-béta, la protéine prion, bien que dépourvues de poly-glutamines.

13. Procédé selon l'une des revendications 1-12, dans lequel la formation de fibres amyloïdes est vérifiée par inspection visuelle du mélange et/ou en déterminant son contenu en protéine après sédimentation par centrifugation de la protéine agrégée.
